# EUROPÄISCHE PATENTANMELDUNG

(11) **EP 3 235 520 A1**
(43) Veröffentlichungstag der Anmeldung: **25.10.2017**
(21) Anmeldenummer: 16166713.4
(22) Anmeldetag: 22.04.2016
(51) Int. Cl.: A61L 15/16, A61L 15/24, A61L 15/26, A61L 15/42, A61L 15/60

(54) **VERFAHREN ZUR HERSTELLUNG EINES PARTIKEL UMFASSENDEN SCHAUMS, SCHAUM MIT HOHER RETENTION UND WUNDAUFLAGE UMFASSEND EINEN SOLCHEN SCHAUM**

(71) Anmelder: Covestro Deutschland AG, 51373 Leverkusen (DE)
(72) Erfinder: WEISER, Marc-Stephan, 51377 Leverkusen (DE); PLUG, Sascha, 51375 Leverkusen (DE); DÖRR, Sebastian, 40593 Düsseldorf (DE); SCHLEMMER, Claudine, 51067 Köln (DE)
(74) Vertreter: Levpat

(57) **Zusammenfassung**

Die vorliegende Erfindung betrifft Verfahren zur Herstellung eines Partikel umfassenden Schaums, in dem eine Mischung umfassend mindestens ein NCO-terminiertes Polyurethan-Prepolymer und Wasser absorbierende Partikel (Superabsorber) wenigstens mit Wasser zusammengeführt werden. Sie betrifft weiterhin einen Schaum mit hoher Retention und eine Wundauflage. Das Verfahren zur Herstellung eines Partikel umfassenden Schaums umfasst die Schritte:
I) Herstellen einer Mischung umfassend mindestens ein NCO-terminiertes Polyurethan-Prepolymer und Wasser absorbierende Partikel;
II) Zusammenführen von mindestens Wasser und der Mischung aus Schritt I).

## Beschreibung

Die vorliegende Erfindung betrifft Verfahren zur Herstellung eines Partikel umfassenden Schaums, in dem eine Mischung umfassend mindestens ein NCO-terminiertes Polyurethan-Prepolymer und Wasser absorbierende Partikel wenigstens mit Wasser zusammengeführt werden. Sie betrifft weiterhin einen Schaum mit hoher Retention und eine Wundauflage.

In der Versorgung von offenen Wunden und insbesondere von chronischen offenen Wunden wie Geschwüren sollte während der exsudativen Phase der Wundheilung die von der Wundstelle abgegebene überschüssige Feuchtigkeit absorbiert werden. Durch einen Sekretstau könnte es ansonsten zu Wundinfektionen kommen. Sehr effektive Mittel zur Absorption von Feuchtigkeit sind superabsorbierende Polymere. Diese sollten jedoch nicht direkt auf der Haut oder gar auf der offenen Wunde aufgetragen werden. Folglich bedarf es der Einhaltung eines Abstands zwischen der Wunde und dem Absorber. Weiterhin wird in der Regel das Absorptionsmittel durch eine weitere Schicht bedeckt, so dass ein Wundpflaster erhalten wird.

WO 95/32860 A1 offenbart schichtförmige, aus wenigstens einer Kunststoffschaum- und/oder Latexschaumschicht sowie partikelförmigen, superabsorbierenden Polymerisaten bestehende Körper zur Absorption von Wasser und wässrigen Flüssigkeiten, die auf, zwischen oder unter der geschäumten Kunststoff- und/oder Latexschicht den Superabsorber in mengenmäßig und/oder örtlich vorgegebener und fixierter flächenmäßiger Anordnung in einem Mengenverhältnis von Kunststoff- und/oder Latexschaum zu Superabsorber von 1:500 bis 50:1 enthalten. Kunststoff-/Latexschaum können Füllstoffe, Pigmente und/oder synthetische Fasern enthalten. Die Schichtkörper weisen eine erhöhte Aufnahmefähigkeit gegenüber Wasser und wässrigen Flüssigkeiten, insbesondere unter Belastung auf. Sie werden hergestellt, indem der Schaum flächenförmig verteilt und der Superabsorber in dem vorgegebenen Mengenverhältnis, gegebenenfalls unter Verwendung einer Schablone aufgebracht und durch Wärmebehandlung fixiert wird. Derartige Schichtkörper werden in Hygieneartikeln, als Komponenten in natürlichen oder künstlichen Böden, als Isoliermaterial für Rohre und Leitungen, vor allem Kabel, und Baukonstruktionen, als flüssigkeitsaufnehmende und -speichernde Komponente in Verpackungsmaterialien sowie als Teil in Bekleidungsstücken verwendet.

EP 2 140 888 A1 betrifft einen Schichtenverbund, geeignet als Wundauflage, umfassend eine Basisschicht, eine auf der Basisschicht aufliegende Absorberschicht sowie eine Deckschicht, wobei die Deckschicht so angebracht ist, dass sie sowohl mit der Basisschicht als auch mit der Absorberschicht verbunden ist und wobei die Basisschicht einen Polyurethanschaum umfasst, der erhalten wird, indem eine Zusammensetzung umfassend eine wässrige, anionisch hydrophilierte Polyurethandispersion (I) aufgeschäumt und getrocknet wird. Die Publikation betrifft weiterhin ein Verfahren zur Herstellung eines solchen Schichtenverbundes sowie dessen Verwendung als Wundauflage, Inkontinenzprodukt und/oder Kosmetikartikel.

WO 2011/023762 A2 beschäftigt sich mit hydrophilen, aliphatischen Polyurethan-Schäumen, die durch Reaktion von speziellen monomerenarmen Prepolymeren und hydrophilen Polyisocyanaten in Gegenwart von Wasser zugänglich sind. Aufgrund ihrer Absorptionseigenschaften sind die Polyurethan-Schäume insbesondere geeignet zur Herstellung von Wundauflagen, kosmetischen Artikeln oder Inkontinenzprodukten.

WO 2011/083144 A1 offenbart ebenfalls hydrophile, aliphatischen Polyurethan-Schäumen, die durch Reaktion von und hydrophilen Polyisocyanaten in Gegenwart von Wasser zugänglich sind. Aufgrund ihrer Absorptionseigenschaften sind die Polyurethan-Schäume insbesondere geeignet zur Herstellung von Wundauflagen, kosmetischen Artikeln oder Inkontinenzprodukten.

WO 2012/055834 A1 betrifft ebenfalls ein Verfahren zur Herstellung von hydrophilen, aliphatischen Polyurethan-Schäumen.

WO 2014/076077 A1 offenbart einen Polyurethanweichschaumstoff enthaltend keimfähige und/oder gekeimte Pflanzensamen. Der Polyurethanweichschaumstoff kann einen Stoff mit hohem Wasserhaltevermögen enthalten, beispielsweise einen Superabsorber auf Polyacrylatbasis.

DE 10 2010 028914 A1 offenbart eine Spritz- und spachtelfähige Masse, enthaltend A) Schaumstoffpartikel, B) 5 bis 100 Gewichtsteile, bezogen auf Feststoff, wässrige Polymerdispersion, C) 0 bis 5 Gewichtsteile eines Schäumungsmittel, D) 0 bis 5 Gewichtsteile eines Verdickers, E) 0 bis 100 Gewichtsteile eines Füllstoffes, F) 0 bis 20 Gewichtsteile eines Flammschutzmittels, G) 10 bis 300 Gewichtsteile Wasser und H) 0,1 bis 10 Gewichtsteile eines Superabsorber, wobei sich die Gewichtsteile jeweils auf 100 Gewichtsteile Schaumstoffpartikel beziehen.

WO 2010/083547 A2 beschreibt ein Schaumstoffelement aus einem Schaumstoff und Partikel aus zumindest einem hydrophilen Mittel, wie Zellulose, Superabsorbentien, wobei das die Partikel enthaltende Schaumstoffelement eine reversible Feuchtigkeitsaufnahmefähigkeit aufweist. Ein Teil der Partikel ist vollständig im Schaumstoff eingebettet und ein weiterer Teil der Partikel ist über eine Oberfläche, wie Zellwände oder Zellstege, des Schaumstoffs vorragend angeordnet.

DE 4124338 A1 offenbart die Herstellung von in Wasser quellbaren Körpern, vorzugsweise zur Verwendung als Dichtungsmasse, gekennzeichnet durch Einbau von Wasser absorbierenden Verbindungen in eine Matrix, die durch Reaktion von an sich bekannten, hydrophilen Isocyanat-Präpolymeren mit bekannten Kettenverlängerungsmitteln zu Polyharnstoffen, entsteht. Die Struktur der Körper kann kompakt oder zellig sein.

Eine wichtige Eigenschaft von Polyurethan-Schäumen in Wundauflagen ist nicht nur das Exsudat aufzunehmen, sondern auch unter Druck zurückzuhalten. Letzteres wird als Retention bezeichnet. Um die Herstellung von Wundauflagen zu vereinfachen und somit auch deren Kosten zu reduzieren wäre es wünschenswert, auf getrennte Schaum- und Absorberbereiche in der Wundauflage zu verzichten. Eine Aufgabe der vorliegenden Erfindung war es, mindestens einen Nachteil des Standes der Technik wenigstens zu einem Teil zu überwinden. Weiterhin hat sich die vorliegende Erfindung die Aufgabe gestellt, die Retention des Wundschaums zu verbessern. Eine weitere Aufgabe der Erfindung war es, eine möglichst hohe Retention des Wundschaums bei mindestens gleichbleibender Absorptionsrate zu erzielen.

Erfindungsgemäß gelöst wird die Aufgabe durch ein Verfahren zur Herstellung eines Partikel umfassenden Schaums, umfassend die Schritte:
I) Herstellen einer Mischung umfassend mindestens ein NCO-terminiertes Polyurethan-Prepolymer und Wasser absorbierende Partikel;
II) Zusammenführen von mindestens Wasser und der Mischung aus Schritt I).

Das NCO-terminierte Polyurethan-Prepolymer und Wasser reagieren unter Bildung von Harnstoffgruppen und CO₂-Abspaltung, so dass ein Polyurethanschaum erhalten wird. Es wurde überraschenderweise gefunden, dass die Wasser absorbierenden Partikel bereits vor dem Zusammenführen mit Wasser in das Prepolymer eingearbeitet werden können und ein Schaum mit hoher Retention erhalten wird, ohne dass die Anwesenheit dieser Feststoff-Partikel die Reaktion stört.

Die Wasser absorbierenden Partikel umfassen ein Material, welches in der Lage ist, Wasser oder andere Flüssigkeiten zu binden. In Frage kommen insbesondere superabsorbierende Polymere (SAP), auch als Superabsorber bezeichnet. Solche Superabsorber sind Materialien, welche ein Mehrfaches ihres Eigengewichts an Wasser absorbieren und unter gewissem Druck zurückhalten können.

Die Superabsorber-Partikel werden selbst dann noch im Schaum als Feststoff-Partikel betrachtet, wenn sie ganz oder teilweise Wasser oder eine wässrige Lösung absorbiert haben.

Der Anteil der Wasser absorbierenden Partikel kann ≥ 1 bis ≤ 100 Gewichts-% (bezogen auf das Gesamtgewicht des Schaums) betragen. Vorzugsweise werden ≥ 2 bis ≤ 50 Gewichts-% Wasser absorbierende Partikel, mehr bevorzugt werden ≥ 2.5 bis ≤ 15 Gewichts-% Wasser absorbierende Partikel eingesetzt.

Unter Superabsorbern sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate sowie vorzugsweise wasserabsorbierende Polymere auf Basis teilneutralisierter Acrylsäure zu verstehen. Ein "Superabsorber" kann auch ein Gemisch stofflich verschiedener einzelner Superabsorber sein.

Üblicherweise liegt das Prepolymer in Schritt I) in flüssiger Form vor. Dann können die festen Partikel direkt in die flüssige Phase eingearbeitet werden. Falls bei Raumtemperatur feste Prepolymere eingesetzt werden, können diese aufgeschmolzen werden. Zur Verringerung der Viskosität kann bei einer Temperatur oberhalb der Raumtemperatur gearbeitet werden. Es ist bevorzugt, dass die in Schritt I) hergestellte Mischung lösungsmittelfrei ist.

In Schritt II) kann sowohl das Wasser oder eine wässrige Mischung zu der in Schritt I) hergestellten Prepolymer/Partikel-Mischung hinzugefügt werden oder umgekehrt die Prepolymer/Partikel-Mischung zu Wasser oder wässriger Mischung. Es ist ebenfalls möglich, dass zwei Stoffströme (Prepolymer/Partikel-Mischung und Wasser/wässrige Mischung) in einem Mischelement zusammengeführt werden.

Nach der Herstellung können die Polyurethan-Schäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden können. In der Regel werden dazu Blockschäume nach gängigen Methoden auf die gewünschte Dicke geschnitten, wodurch flächige Materialien mit einer Dicke von typischerweise von ≥ 10 µm bis ≤ 5 cm erhalten werden. Mit Hilfe geeigneter Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier oder Textil, erhalten werden.

Ausführungsformen und weitere Aspekte der vorliegenden Erfindung werden nachfolgend beschrieben. Sie können beliebig kombiniert werden, sofern sich aus dem Kontext nicht eindeutig das Gegenteil ergibt.

In einer Ausführungsform des erfindungsgemäßen Verfahrens ist das NCO-terminierte Polyurethan-Prepolymer erhältlich aus der Reaktion einer Reaktionsmischung umfassend ein aliphatisches Polyisocyanat und ein Polyoxyethylengruppen enthaltendes Polyol. Hinsichtlich der Polyisocyanate sind Hexamethylendiisocyanat und Isophorondiisocyanat bevorzugt, bei den Polyolen Copolymere aus Ethylenoxid und Propylenoxid mit einem Ethylenoxidgehalt, bezogen auf die Gesamtmenge der enthaltenen Oxyalkylengruppen, von 60 bis 85 mol-%.

In einer weiteren Ausführungsform des erfindungsgemäßen Verfahrens wird Schritt II) weiterhin in Gegenwart einer Base durchgeführt. Vorzugsweise weist die Base einen pK_{b}-Wert von ≥ 6, mehr bevorzugt ≥ 7 auf. Besonders bevorzugt ist eine CO₂ freisetzende Base wie Hydrogencarbonat.

In einer weiteren Ausführungsform des erfindungsgemäßen Verfahrens weisen die Wasser absorbierenden Partikel eine Zentrifugen-Retentions-Kapazität von ≥ 5 g/g auf. Die Zentrifugen-Retentions-Kapazität (Centrifuge Retention Capacity, CRC) beträgt vorzugsweise mindestens 10 g/g und in besonders bevorzugter Form mindestens 15 g/g.

Die Centrifuge Retention Capacity wird gemäß der von der EDANA (European Disposables and Nonwovens Association, Avenue Eugene Plasky 157, 1030 Brüssel, Belgien) empfohlenen und anhand der dort erhältlichen Testmethode Nr. 441.2-02 "Centrifuge Retention Capacity" bestimmt.

In einer weiteren Ausführungsform des erfindungsgemäßen Verfahrens weist das NCO-terminierte Polyurethan-Prepolymer einen Gewichtsanteil an niedermolekularen, aliphatischen Diisocyanaten mit einer Molmasse von ≥ 140 bis ≤ 278 g/mol, bevorzugt ≥ 168 bis ≤ 258 g/mol von ≤ 1.0 Gew.-%, bezogen auf das Prepolymer, auf und ist als Komponente A) erhältlich durch Umsetzung von
A1) niedermolekularen, aliphatischen Diisocyanaten einer Molmasse von ≥ 140 bis ≤ 278 g/mol, bevorzugt ≥ 168 bis ≤ 258 g/mol, mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen.

In einer weiteren bevorzugten Ausführungsform des erfindungsgemäßen Verfahrens umfasst die Mischung in Schritt I) weiterhin:
H1) ein oder mehrere niedermolekulare, aliphatische Diisocyanate mit einer Molmasse von ≥ 140 bis ≤ 278 g/mol, bevorzugt ≥ 168 bis ≤ 258 g/mol und/oder daraus herstellbare Polyisocyanate mit einer Isocyanatfunktionalität von ≥ 2 bis ≤ 6;
   und/oder
H2) ein oder mehrere monofunktionelle Polyalkylenoxide einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
und/oder
eine hydrophile Isocyanatkomponente, welche durch die Umsetzung von unter H1) und/oder A) genannten Komponenten mit unter H2) genannten Komponenten erhältlich ist.

Die Mischung in Schritt I) umfasst bevorzugt:
A) isocyanatfunktionelle Prepolymere mit einem Gewichtsanteil an niedermolekularen, aliphatischen Diisocyanaten mit einer Molmasse von ≥ 140 bis ≤ 278 g/mol, bevorzugt ≥ 168 bis ≤ 258 g/mol von ≤ 1.0 Gew.-% bezogen auf das Prepolymer, erhältlich durch Umsetzung von
A1) niedermolekularen, aliphatischen Diisocyanaten einer Molmasse von ≥ 140 bis ≤ 278 g/mol, bevorzugt ≥ 168 bis ≤ 258 g/mol mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen,
B) gegebenenfalls heterocyclische, 4-Ring oder 6-Ring-Oligomere von niedermolekularen, aliphatischen Diisocyanaten mit einer Molmasse von ≥ 140 bis ≤ 278 g/mol, bevorzugt ≥ 168 bis ≤ 258 g/mol,
D) gegebenenfalls Katalysatoren,
E) gegebenenfalls Alkalimetallsalze schwacher anorganischer Säuren,
F) gegebenenfalls Tenside,
G) gegebenenfalls ein- oder mehrwertige Alkohole,
H) die folgenden Komponenten:
   H1) ein oder mehrere niedermolekulare, aliphatische Diisocyanate einer Molmasse von ≥ 140 bis ≤ 278 g/mol, bevorzugt ≥ 168 bis ≤ 258 g/mol und/oder daraus herstellbare Polyisocyanate mit einer Isocyanatfunktionalität von ≥ 2 bis ≤ 6;
      und/oder
   H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
und/oder
eine hydrophile Isocyanatkomponente, welche durch die Umsetzung von unter H1) und/oder A) genannten Komponenten mit unter H2) genannten Komponenten erhältlich ist.

Bevorzugt weisen die eingesetzten Prepolymere A) einen Restmonomergehalt von unter 0.5 Gew.-% bezogen auf das Prepolymer auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A1) im Überschuss und mit anschließender, bevorzugt destillativer, Abtrennung nicht umgesetzter Monomere.

Bei der Herstellung der isocyanatfunktionellen Prepolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 2 bis 20 Mol, bevorzugt mit 2 bis 10 Mol und besonders bevorzugt 5 bis 10 Mol NCO-Gruppen des niedermolekularen, aliphatischen Diisocyanats A1) kommen.

Die Herstellung der unter H) genannten hydrophilen Polyisocyanate erfolgt typischerweise durch Umsetzung von 1 Mol OH-Gruppen der monofunktionellen Polyalkylenoxidkomponente H2) mit 1.25 bis 15 Mol, bevorzugt mit 2 bis 10 Mol und besonders bevorzugt 2 bis 6 Mol NCO-Gruppen eines Polyisocyanates H1) mit einer Isocyanatfunktionalität von 2 bis 6, basierend auf aliphatischen Diisocyanaten. Beispielhaft für derartige Polyisocyanate H1) sind Biuret-Strukturen, Isocyanurate bzw. Uretdione basierend auf aliphatischen Diisocyanaten. Dabei werden das Polyisocyanat H1) und das Polyalkylenoxid H2) bevorzugt über eine Urethangruppe bzw. eine Harnstoffgruppe miteinander verknüpft, wobei besonders die Verknüpfung über Urethangruppen bevorzugt ist.

Der NCO-Gehalt der isocyanatfunktionellen Prepolymere A) beträgt bevorzugt 1.5 bis 4.5 Gew.-%, besonders bevorzugt 1.5 bis 3.5 Gew.-% und ganz besonders bevorzugt 1.5 bis 3.0 Gew.-%.

Beispiele für niedermolekulare, aliphatische Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol-%, bevorzugt von 60 bis 85 mol-%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide der Komponente A2) besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) OH-Funktionalitäten von 2 bis 6, bevorzugt von 3 bis 6, besonders bevorzugt von 3 bis 4.

Gegebenenfalls einzusetzende Verbindungen der Komponente B) sind heterocyclische, 4-Ring oder 6-Ring-Oligomere von niedermolekularen, aliphatischen Diisocyanaten mit einer Molmasse von 140 bis 278 g/mol, bevorzugt ≥ 168 bis ≤ 258 g/mol, wie Isocyanurate, Iminooxadiazindione oder Uretdione der vorgenannten niedermolekularen, aliphatischen Diisocyanate.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation. Vor, während und nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-tert-butylkresol oder Tocopherol zugesetzt werden. Der NCO-Gehalt der hydrophilen Polyisocyanate H) beträgt bevorzugt 0.3 bis 20 Gew.-%, besonders bevorzugt 2 bis 10 Gew.-% und ganz besonders bevorzugt 3 bis 6 Gew.-%.

Zur Beschleunigung der Harnstoff - bzw. Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1,8-Diazabi-cyclo[5.4.0]undecen-7 (DBU), 1,5-Diazabicyclo[4.3.0]nonen-5 (DBN), 1,4-Diazabicy-clo[3.3.0]octen-4 (DBO), N-Ethylmorpholin (NEM), Triethylendiamin (DABCO), Pentamethylguanidin (PMG), Tetramethylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente D). Bevorzugt ist auch die Verwendung von 1,8-Diazabicyclo[5.4.0]undecen-7 (DBU).

Es kann aber auch auf Katalysatoren verzichtet werden; dies ist bevorzugt.

Als Komponente E) werden Alkalimetallsalze schwacher anorganischer Säuren eingesetzt. Hierunter werden Alkalimetallsalze von anorganischen Säuren verstanden, deren korrespondierende freie Säuren in Wasser bei 25 °C einen pKs-Wert von ≥ 4.0 aufweisen. Beispiele besonders geeigneter Alkalimetallsalze schwacher anorganischer Säuren sind Kaliumhydroxid, Natriumhydroxid, Kaliumcarbonat, Natriumcarbonat und Natriumhydrogencarbonat, wobei auch beliebige Mischungen dieser Salze mit eingeschlossen sind.

Zur Verbesserung der Schaumbildung, Schaumstabilität oder der Eigenschaften des resultierenden Polyurethan-Schaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethan-Schaums Verbindungen der Komponente G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Bei der Herstellung der unter H) aufgeführten hydrophilen Polyisocyanate wird das Verhältnis der monofunktionellen Polyalkylenoxide H2) zu den niedermolekularen, aliphatischen Diisocyanaten H1) bevorzugt so eingestellt, dass auf 1 Mol OH-Gruppen der monofunktionellen Polyalkylenoxide 1.25 bis 15 Mol, bevorzugt 2 bis 10 Mol und besonders bevorzugt 2 bis 6 Mol NCO-Gruppen des niedermolekularen, aliphatischen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Beispiele für niedermolekulare, aliphatische Diisocyanate der Komponente H1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat. Beispiele für höhermolekulare Polyisocyanate H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H1) höhermolekulare Verbindungen mit Biuret-, Iminooxadiazindion-, Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxide H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden H2) durch Alkoxylierung geeigneter Startermoleküle ist literaturbekannt (z.B. Ullmanns Enzyklopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet. Die monofunktionellen Polyalkylenoxide H2) besitzen typischerweise zahlenmittlere Molekulargewichte von 220 bis 3700 g/mol, bevorzugt von 500 bis 2800 g/mol.

Die monofunktionellen Polyalkylenoxide H2) besitzen bevorzugt eine OH-Gruppe als isocyanatreaktive Gruppe.

Typischerweise werden die Komponenten A) bis H) in folgenden Mengen eingesetzt (zur Reaktion mit 0.1 bis 200 Gewichtsteilen Wasser in Schritt II) des erfindungsgemäßen Verfahrens):
100 Gewichtsteile isocyanatfunktioneller Prepolymere A)
0 bis 30 Gewichtsteile heterocyclischer Oligomere B)
0 bis 1 Gewichtsteile an Katalysatoren D)
0 bis 5 Gewichtsteile Alkalimetallsalze schwacher anorganische Säuren E)
0 bis 10 Gewichtsteile Tenside F)
0 bis 20 Gewichtsteile Alkohole G)
5 bis 250 Gewichtsteile einer oder mehrerer unter H) genannten Komponenten.

Der Anteil der Wasser absorbierenden Partikel kann ≥ 1 bis ≤ 100 Gewichts-% (bezogen auf das Gesamtgewicht des erhaltenen Schaums) betragen. Vorzugsweise werden ≥ 2 bis ≤ 50 Gewichts-% Wasser absorbierende Partikel, mehr bevorzugt werden ≥ 2.5 bis ≤ 15 Gewichts-% Wasser absorbierende Partikel eingesetzt.

Bevorzugt werden die Komponenten A) bis H) in folgenden Mengen eingesetzt (zur Reaktion mit 0.1 bis 100 Gewichtsteilen Wasser in Schritt II) des erfindungsgemäßen Verfahrens):
100 Gewichtsteile isocyanatfunktioneller Prepolymere A)
1 bis 30 Gewichtsteile heterocyclischer Oligomere B)
0 bis 1 Gewichtsteile an Katalysatoren D)
0.01 bis 5 Gewichtsteile Alkalimetallsalze schwacher anorganische Säuren E)
0 bis 5 Gewichtsteile Tenside F)
0 bis 10 Gewichtsteile Alkohole G)
10 bis 100 Gewichtsteilen einer oder mehrerer unter H) genannten Komponenten.

Der Anteil der Wasser absorbierenden Partikel kann ≥ 1 bis ≤ 100 Gewichts-% (bezogen auf das Gesamtgewicht des erhaltenen Schaums) betragen. Vorzugsweise werden ≥ 2 bis ≤ 50 Gewichts-% Wasser absorbierende Partikel, mehr bevorzugt werden ≥ 2.5 bis ≤ 15 Gewichts-% Wasser absorbierende Partikel eingesetzt.

Besonders bevorzugt werden die Komponenten A) bis H) in folgenden Mengen eingesetzt (zur Reaktion mit 0,1 bis 60 Gewichtsteilen Wasser in Schritt II) des erfindungsgemäßen Verfahrens):
100 Gewichtsteile isocyanatfunktioneller Prepolymere A)
5 bis 15 Gewichtsteile heterocyclischer Oligomere B)
0 bis 0,5 Gewichtsteile an Katalysatoren D)
0.1 bis 1 Alkalimetallsalze schwacher anorganische Säuren E)
0 Gewichtsteile Tenside F)
0 Gewichtsteile Alkohole G)
20 bis 80 Gewichtsteilen einer oder mehrerer unter H) genannten Komponenten.

Der Anteil der Wasser absorbierenden Partikel kann ≥ 1 bis ≤ 100 Gewichts-% (bezogen auf das Gesamtgewicht des erhaltenen Schaums) betragen. Vorzugsweise werden ≥ 2 bis ≤ 50 Gewichts-% Wasser absorbierende Partikel, mehr bevorzugt werden ≥ 2.5 bis ≤ 15 Gewichts-% Wasser absorbierende Partikel eingesetzt.

In einer bevorzugten Ausführungsform des erfindungsgemäßen Verfahrens werden in der Mischung in Schritt I) auf 100 Gewichtsteile NCO-terminierte Prepolymere ≥ 5 bis ≤ 250 Gewichtsteile:
hydrophile Polyisocyanatkomponente H1) und/oder
das Reaktionsprodukt von H1) mit H2) und/oder
das Reaktionsprodukt von H2) mit A)
eingesetzt und in Schritt II) ≥ 1 bis ≤ 200 Gewichtsteile Wasser, bezogen auf 100 Gewichtsteile NCO-terminierte Prepolymere der Mischung in Schritt I), eingesetzt.

In einer bevorzugten Ausführungsform des erfindungsgemäßen Verfahrens weisen die Wasser absorbierenden Partikel einen d₉₀-Wert der Teilchengrößenverteilung von ≤ 1000 µm auf. Die Teilchengrößenverteilung kann anhand der Norm DIN EN 933 bestimmt werden. Vorzugsweise beträgt der d₉₀-Wert ≥ 10 bis ≤ 700 µm, mehr bevorzugt ≥ 50 bis ≤ 500 µm, besonders bevorzugt ≥ 100 bis ≤ 400 µm.

In einer weiteren bevorzugten Ausführungsform des erfindungsgemäßen Verfahrens weisen die Wasser absorbierenden Partikel wenigstens auf ihrer Oberfläche gegenüber NCO-Gruppen reaktive funktionelle Gruppen auf. Solche funktionelle Gruppen sind insbesondere Zerewitinoff-aktive H-Atome einschließlich COOH-Gruppen.

In einer weiteren bevorzugten Ausführungsform des erfindungsgemäßen Verfahrens sind die Wasser absorbierenden Partikel Polyacrylat- und/oder Carboxymethylcellulose-Partikel. Vorzugsweise handelt es sich dem Polyacrylat um ein Copolymer aus Acrylsäure und Natriumacrylat oder ein vernetztes Copolymer von Acrylsäuren mit bi- und/oder polyfunktionellen Monomeren. Beispiele für bi- und/oder polyfunktionelle Monomere sind Polyallylglucosen. Hinsichtlich der Carboxymethylcellulose sind Alkalisalze wie Natriumsalze bevorzugt, welche aus der Reaktion von Cellulose mit dem Natriumsalz der Chloressigsäure erhältlich sind. Es ist auch möglich, dass die Carboxymethylcellulose quervernetzt ist, beispielsweise durch Ester- oder Etherbindungen.

In einer weiteren bevorzugten Ausführungsform des erfindungsgemäßen Verfahrens werden in der Mischung in Schritt I) auf 100 Gewichtsteile NCO-terminierte Prepolymere ≥ 1 bis ≤ 100 Gewichtsteile Wasser absorbierende Partikel eingesetzt. Vorzugsweise werden ≥ 2 bis ≤ 50 Gewichtsteile Wasser absorbierende Partikel, mehr bevorzugt werden ≥ 2.5 bis ≤ 15 Gewichtsteile Wasser absorbierende Partikel eingesetzt.

Die Umsetzung mit Wasser in Schritt II) kann in Gegenwart von Katalysatoren für die Harnstoffbildung aus Isocyanaten und/oder Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Bevorzugt beinhaltet das Wasser ein oder mehrere Alkalimetallsalze, wie sie als Komponente E) für das Prepolymer beschrieben wurden. Bevorzugt beinhaltet das Wasser Alkalimetallsalze in einer Menge in einem Bereich von 0,1 bis 10 Gew.-%, oder bevorzugt in einem Bereich von 0,5 bis 5 Gew.-%, oder bevorzugt in einem Bereich von 0,5 bis 3 Gew.-%, bezogen auf die Gesamtmenge des in Schritt II) zugesetzten Wassers.

Weiterhin beinhaltet das Wasser bevorzugt ein oder mehrere Tenside, wie sie als Komponente F) für das Prepolymer beschrieben wurden. Bevorzugt beinhaltet das Wasser Tenside in einer Menge in einem Bereich von 0,1 bis 10 Gew.-%, oder bevorzugt in einem Bereich von 0,5 bis 5 Gew.-%, oder bevorzugt in einem Bereich von 0,5 bis 3 Gew.-%, bezogen auf die Gesamtmenge des in Schritt II) zugesetzten Wassers.

Die Umsetzung mit Wasser erfolgt typischerweise bei 10 bis 140 °C, bevorzugt 60 bis 100 °C.

In einer weiteren bevorzugten Ausführungsform des erfindungsgemäßen Verfahrens wird Schritt II) ≥ 1 Sekunde bis ≤ 60 Minuten nach Schritt I) durchgeführt.

In einer weiteren bevorzugten Ausführungsform des erfindungsgemäßen Verfahrens umfasst dieses weiterhin die Schritte:
III) Mischen der nach Schritt II) erhaltenen Mischung;
IV) Auftragen der nach Schritt III) erhaltenen Mischung auf ein Substrat;
V) Trocknen der auf dem Substrat nach Schritt IV) befindlichen Mischung.

Nach dem Zusammenführen der in Schritt I) erhaltenen Mischung mit Wasser oder einer wässrigen Mischung in Schritt II) wird eine neue Mischung erhalten. Diese wird in Schritt III) miteinander vermischt. Durch das Mischen wird bevorzugt eine homogene Mischung erhalten. Unter homogener Mischung wird eine Abweichung der Konzentration jeder der Komponenten, die in Schritt II) zusammengeführt werden von bevorzugt weniger als 10 Gew.-%, oder bevorzugt von weniger als 5 Gew.-%, oder bevorzugt von weniger als 3 Gew.-%, bezogen auf die Gesamtmenge der Mischung in benachbarten Volumenelementen von 5 ml verstanden. Als Mischaggregat können alle geeigneten Mischaggregate eingesetzt werden. Vorzugsweise werden Rührwerksmischer oder Statikmischer oder Kombinationen daraus eingesetzt.

Die Polyurethan-Schäume können nach ihrer Trocknung eine mikroporöse, zumindest teilweise offenporige Struktur mit miteinander kommunizierenden Zellen aufweisen. Die Dichte der getrockneten Schäume liegt dabei typischerweise unterhalb von 0.4 g/cm³, bevorzugt ist sie geringer als 0.35 g/cm³, besonders bevorzugt ≥ 0.01 g/cm³ bis ≤ 0.3 g/cm³ und liegt ganz besonders bevorzugt bei ≥ 0.07 g/cm³ bis ≤ 0.25 g/cm³.

Die Polyurethan-Schaumschichten haben nach dem Trocknen typischerweise eine Dicke von ≥ 0.1 mm bis ≤ 50 mm, bevorzugt ≥ 0.5 mm bis ≤ 20 mm, besonders bevorzugt ≥ 1 mm bis ≤ 10 mm, ganz besonders bevorzugt ≥ 1.5 mm bis ≤ 5 mm.

Der erhaltene Schaum wird beim Aufschäumen oder unmittelbar danach auf ein Substrat aufgetragen und getrocknet. Als Substrate geeignet sind insbesondere Papiere oder Folien, die ein einfaches Ablösen der Wundauflage vor ihrem Einsatz zur Abdeckung einer verletzten Stelle ermöglichen. Weiterhin kann das Substrat eine spätere Deckschicht einer Wundauflage sein.

Der Auftrag kann beispielsweise durch Gießen oder Rakeln erfolgen, aber auch andere an sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20 °C beobachtet. Für eine schnellere Trocknung und Fixierung der Schäume werden jedoch bevorzugt Temperaturen oberhalb von 30 °C benutzt. Bei der Trocknung sollten jedoch Temperaturen von 200 °C, bevorzugt 180 °C, besonders bevorzugt 150 °C nicht überschritten werden, da es anderenfalls zu unerwünschter Vergilbung und/oder Zersetzung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlern. Auch die Trocknung durch Führen des beschichteten Substrates über geheizte Oberflächen, zum Beispiel Walzen, ist möglich.

Der Auftrag sowie die Trocknung können jeweils diskontinuierlich oder kontinuierlich durchgeführt werden, bevorzugt ist jedoch ein kontinuierliches Verfahren.

In einer weiteren bevorzugten Ausführungsform des erfindungsgemäßen Verfahrens herrscht in Schritt V) eine Temperatur von ≤ 110 °C. Vorzugsweise beträgt die Temperatur ≥ 60 °C bis ≤ 105 °C.

Ein weiterer Gegenstand der vorliegenden Erfindung ist ein Schaum, welcher eine Retention von ≥ 50% aufweist, wobei die Retention wie folgt bestimmt wird:
- ein Schaumstück der Größe 5 x 5 cm wird nach vollständiger Absorption (Prüflösung A, hergestellt nach DIN EN 13726-1) mit einer Pinzette angehoben und für 30 Sekunden abtropfen gelassen und gewogen;
- das Schaumstück wird auf ein Metallpodest gelegt und mit einem Gewicht von 6 kg für 20 Sekunden beschwert;
- das Gewicht wird entfernt und nach Entfernen des Gewichtes wird das Schaumstück erneut gewogen;
- die verbliebene Feuchtigkeitsmenge wird im Vergleich prozentual zur maximalen Absorption bestimmt.

Vorzugsweise beträgt die Retention des Schaums ≥ 55%, mehr bevorzugt ≥ 60%.

Der erfindungsgemäße Schaum findet bevorzugt Verwendung als Bestandteil von Wundauflagen, Inkontinenzprodukten und/oder Kosmetikartikeln. Inkontinenzprodukte können beispielsweise Windeln für Säuglinge, Kinder und Erwachsene sein. Kosmetikartikel können zum Beispiel Reinigungsartikel sein. Bevorzugt ist die Verwendung als Wundauflage. Dann kann der Schaum als "Wundschaum" bezeichnet werden.

In einer bevorzugten Ausführungsform des erfindungsgemäßen Schaums weist dieser weiterhin ein maximales Absorptionsvermögen für Wasser von ≥ 1500% auf. Die maximale Absorption des Schaumes kann gemäß DIN EN 13726-1 an einem Schaumstück der Größe 5 x 5 cm bestimmt werden. Vorzugsweise beträgt die maximale Absorption ≥ 1600%, mehr bevorzugt ≥ 1700%, besonders bevorzugt ≥ 1800%, mehr bevorzugt ≥ 1900% und insbesondere bevorzugt ≥ 2000%.

In einer weiteren bevorzugten Ausführungsform des erfindungsgemäßen Schaums umfasst dieser weiterhin Wasser absorbierende Partikel in einem Anteil von ≥ 1 bis ≤ 100 Gewichts-% (bezogen auf das Gesamtgewicht des Schaums). Vorzugsweise werden ≥ 2 bis ≤ 50 Gewichts-% Wasser absorbierende Partikel, mehr bevorzugt werden ≥ 2.5 bis ≤ 15 Gewichts-% Wasser absorbierende Partikel eingesetzt.

In einer weiteren bevorzugten Ausführungsform des erfindungsgemäßen Schaums ist der Schaum durch ein erfindungsgemäßes Verfahren erhältlich. Zur Vermeidung von Wiederholungen wird auf die vorangegangenen Ausführungen zum Verfahren verwiesen.

Die vorliegende Erfindung betrifft weiterhin eine Wundauflage, umfassend eine Deckschicht und eine mit der Deckschicht verbundene Schaumschicht, wobei die Schaumschicht zumindest teilweise durch einen erfindungsgemäßen Schaum gebildet wird. Falls eine Trennung der Wunde von der Schaumschicht gewünscht ist, kann noch eine Wundkontaktschicht vorgesehen sein. Die Wundauflage weist dann die Schichtenfolge Deckschicht-Schaumschicht-Wundkontaktschicht auf.

### Beispiele

Die Erfindung wird anhand der nachfolgenden Beispiele näher erläutert, ohne jedoch darauf beschränkt zu sein.

Sofern nicht anders angegeben, wurden die Versuche bei 23 °C durchgeführt. Die %-Angaben beziehen sich auf Gewichts-%, sofern nicht anders angegeben.

### Eingesetzte Substanzen:

Baymedix FP505 ist ein Produkt der Covestro Deutschland AG, Leverkusen, Deutschland (aliphatisches, hydrophiles Prepolymer).

Akucell AF2881 ist ein Handelsprodukt der Akzo Nobel Functional Chemicals AB, Stenungsund, Schweden (Superabsorber auf Basis von Carboxymethylcellulose).

Favor PAC 300 ist ein Handelsprodukt der Evonik Industries AG, Krefeld (Superabsorber auf Basis von vernetztem Polyacrylat). Bei diesem Superabsorber liegt die Wasseraufnahme laut Datenblatt bei ca. 77 g/g und die Aufnahme von 0.9 %-ige NaCl-Lösung bei ca. 44 g/g.

Natriumhydrogencarbonat wurde von Sigma/Aldrich Chemie GmbH, Steinheim, Deutschland erhalten.

Zitronensäure-Monohydrat wurde von Acros Organics, Geel, Belgien erhalten.

Pluronic PE6800 ist ein Handelsprodukt der BASF SE, Ludwigshafen, Deutschland (nichtionisches Tensid, EO/PO-Block-Copolymer mit 80 Gewichts-% EO).

### Methoden

Die maximale Absorption des Schaumes wurde gemäß DIN EN 13726-1 an einem Schaumstück der Größe 5 x 5 cm bestimmt.

Für die Bestimmung der Retention wird ein Schaumstück der Größe 5 x 5 cm nach vollständiger Absorption mit einer Pinzette angehoben und für 30 Sekunden abtropfen gelassen und gewogen. Danach wird das Schaumstück auf ein Metallpodest gelegt und mit einem Gewicht von 6 kg für 20 Sekunden beschwert. Nach Entfernen des Gewichtes wird das Schaumstück erneut gewogen und die verbliebene Feuchtigkeitsmenge im Vergleich prozentual zur maximalen Absorption bestimmt.

### Vergleichsbeispiel 1 (Schaum ohne SAP):

125 g Baymedix FP505 wurden mit 25 g einer wässrigen Lösung aus 1,32% Natriumhydrogencarbonat, 4.8% Pluronic PE6800 und 0.4% Zitronensäure-Monohydrat unter heftigem Rühren mit Hilfe eines Rührwerks mit Ankerrührblatt bei 930 U/min. vermischt und danach auf ein Trennpapier des Typs Y 05200 der Felix Schöller Group, Osnabrück mit einem Nassspalt von 1.5 mm aufgerakelt. Danach wurde die Oberfläche der Reaktionsmischung mit einer genadelten Variante desselben Papiers abgedeckt und der Verbund für 5 min. im Konvektionsofen bei 80 °C getrocknet.

Der trockene Schaum zeigte eine maximale Absorption von 2000% und eine Retention von 42%.

### Beispiel 1:

Herstellung wie im Vergleichsbeispiel 1 außer:

125 g Baymedix FP505 wurden mit 6.25 g Akucell AF2881 für 1 min. bei 2750 U/min mit Hilfe eines Speedmixers vermischt. Diese Mischung wurde dann nach einer weiteren Vorrührzeit von 20 s mit Hilfe eines KPG-Rührers mit Ankerrührblatt bei 930 U/min mit der wässrigen Lösung gemischt und für 7 s weiter gerührt.

Der trockene Schaum zeigte eine maximale Absorption von 2000 % und eine Retention von 70%.

### Beispiel 2:

Herstellung wie im Vergleichsbeispiel 1 außer:

125 g Baymedix FP505 wurden mit 6.25 g Favor PAC 300, welches vorher in einem Mörser zermahlen wurde, für 1 min. bei 2750 U/min mit Hilfe eines Speedmixers vermischt. Diese Mischung wird dann nach einer weiteren Vorrührzeit von 20 s mit Hilfe eines KPG-Rührers mit Ankerrührblatt bei 930 U/min mit der wässrigen Lösung gemischt und für 7s weiter gerührt.

Der trockene Schaum zeigte eine maximale Absorption von 1900 % und eine Retention von 50%.

## Patentansprüche

1. Verfahren zur Herstellung eines Partikel umfassenden Schaums, umfassend die Schritte:
I) Herstellen einer Mischung umfassend mindestens ein NCO-terminiertes Polyurethan-Prepolymer und Wasser absorbierende Partikel;
II) Zusammenführen von mindestens Wasser und der Mischung aus Schritt I).

2. Verfahren gemäß Anspruch 1, wobei das NCO-terminierte Polyurethan-Prepolymer erhältlich ist aus der Reaktion einer Reaktionsmischung umfassend ein aliphatisches Polyisocyanat und ein Polyoxyethylengruppen enthaltendes Polyol.

3. Verfahren gemäß Anspruch 1 oder 2, wobei Schritt II) weiterhin in Gegenwart einer Base durchgeführt wird.

4. Verfahren gemäß einem der Ansprüche 1 bis 3, wobei die Wasser absorbierenden Partikel eine Zentrifugen-Retentions-Kapazität von ≥ 5 g/g aufweisen.

5. Verfahren gemäß einem der Ansprüche 1 bis 4, wobei das NCO-terminierte Polyurethan-Prepolymer einen Gewichtsanteil an niedermolekularen, aliphatischen Diisocyanaten mit einer Molmasse von ≥ 140 bis ≤ 278 g/mol von ≤ 1.0 Gew.-%, bezogen auf das Prepolymer, aufweist und als Komponente A) erhältlich ist durch Umsetzung von
A1) niedermolekularen, aliphatischen Diisocyanaten einer Molmasse von ≥ 140 bis ≤ 278 g/mol mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen.

6. Verfahren gemäß einem der Ansprüche 1 bis 5, wobei die Mischung in Schritt I) weiterhin umfasst:
H1) ein oder mehrere niedermolekulare, aliphatische Diisocyanate mit einer Molmasse von ≥ 140 bis ≤ 278 g/mol und/oder daraus herstellbare Polyisocyanate mit einer Isocyanatfunktionalität von ≥ 2 bis ≤ 6;
und/oder
H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
und/oder
eine hydrophile Isocyanatkomponente, welche durch die Umsetzung von unter H1) und/oder A) genannten Komponenten mit unter H2) genannten Komponenten erhältlich ist

7. Verfahren gemäß Anspruch 6, wobei in der Mischung in Schritt I) auf 100 Gewichtsteile NCO-terminierte Prepolymere ≥ 5 bis ≤ 250 Gewichtsteile:
hydrophile Isocyanatkomponente H1) und/oder
das Reaktionsprodukt von H1) mit H2) und/oder
das Reaktionsprodukt von H2) mit A)
eingesetzt werden und in Schritt II) ≥ 1 bis ≤ 200 Gewichtsteile Wasser, bezogen auf 100 Gewichtsteile NCO-terminierte Prepolymere der Mischung in Schritt I), eingesetzt werden.

8. Verfahren gemäß einem der Ansprüche 1 bis 7, wobei die Wasser absorbierenden Partikel Polyacrylat- und/oder Carboxymethylcellulose-Partikel sind.

9. Verfahren gemäß einem der Ansprüche 1 bis 8, wobei in der Mischung in Schritt I) auf 100 Gewichtsteile NCO-terminierte Prepolymere ≥ 1 bis ≤ 100 Gewichtsteile Wasser absorbierende Partikel eingesetzt werden.

10. Verfahren gemäß einem der Ansprüche 1 bis 9, weiterhin umfassend die Schritte:
III) Mischen der nach Schritt II) erhaltenen Mischung;
IV) Auftragen der nach Schritt III) erhaltenen Mischung auf ein Substrat;
V) Trocknen der auf dem Substrat nach Schritt IV) befindlichen Mischung.

11. Ein Schaum, **dadurch gekennzeichnet, dass** der Schaum eine Retention von ≥ 50% aufweist, wobei die Retention wie folgt bestimmt wird:
- ein Schaumstück der Größe 5 x 5 cm wird nach vollständiger Absorption (Prüflösung A, hergestellt nach DIN EN 13726-1) mit einer Pinzette angehoben und für 30 Sekunden abtropfen gelassen und gewogen;
- das Schaumstück wird auf ein Metallpodest gelegt und mit einem Gewicht von 6 kg für 20 Sekunden beschwert;
- das Gewicht wird entfernt und nach Entfernen des Gewichtes wird das Schaumstück erneut gewogen;
- die verbliebene Feuchtigkeitsmenge wird im Vergleich prozentual zur maximalen Absorption bestimmt.

12. Der Schaum gemäß Anspruch 11, wobei der Schaum ein maximales Absorptionsvermögen für Wasser von ≥ 1500% aufweist.

13. Der Schaum gemäß Anspruch 11 oder 12, weiterhin umfassend Wasser absorbierende Partikel in einem Anteil von ≥ 1 bis ≤ 100 Gewichts-%.

14. Der Schaum gemäß einem der Ansprüche 11 bis 13, wobei der Schaum durch ein Verfahren gemäß einem der Ansprüche 1 bis 10 erhältlich ist.

15. Eine Wundauflage, umfassend eine Deckschicht und eine mit der Deckschicht verbundene Schaumschicht, **dadurch gekennzeichnet, dass** die Schaumschicht zumindest teilweise durch einen Schaum gemäß einem der Ansprüche 11 bis 14 gebildet wird.
